# EUROPÄISCHE PATENTSCHRIFT

(11) **EP 2 564 889 B1**
(45) Veröffentlichungstag und Bekanntmachung des Hinweises auf die Patenterteilung: **04.11.2020**
(21) Anmeldenummer: 12178816.0
(22) Anmeldetag: 01.08.2012
(51) Int. Cl.: A61N 1/05, A61M 25/09

(54) **Führungsdraht-Einführhilfe**
Guide wire insertion aid
Aide à l'introduction d'un fil de guidage

(30) Priorität: 31.08.2011 US 201161529261 P
(43) Veröffentlichungstag der Anmeldung: 06.03.2013
(73) Patentinhaber: BIOTRONIK SE & Co. KG, 12359 Berlin (DE)
(72) Erfinder: Bartels, Klaus, 10115 Berlin (DE); Schippel, Mark, 15834 Rangsdorf (DE); Eichberg, Roland, 10409 Berlin (DE); Voigt, Siegfried, 12161 Berlin (DE)
(74) Vertreter: Galander, Marcus

(56) Entgegenhaltungen:
- US-A- 6 110 146
- US-A1- 2004 073 193
- US-A1- 2006 116 691
- US-A1- 2008 082 051
- US-A1- 2010 174 139

## Beschreibung

Die Erfindung betrifft eine Führungsdraht-Einführhilfe zum Aufsetzen auf ein proximales Ende eines Katheters oder einer implantierbaren Elektrodenleitung und zur Erleichterung der Einführung eines Führungsdrahtes.

Die Handhabung von Kathetern oder katheterähnlichen Instrumenten und insbesondere von implantierbaren Elektrodenleitungen, wie sie in Verbindung mit Herzschrittmachern oder implantierbaren Kardiovertern eingesetzt werden, ist in der klinischen Praxis weit verbreitet. In anspruchsvolleren Anwendungen erfolgt der Einsatz üblicherweise in Verbindung mit Führungsdrähten, die eine präzise Führung und Positionierung des jeweiligen Katheters bzw. der Elektrodenleitung auch dann erlauben, wenn verzweigte Gefäße oder kompliziert geformte Hohlräume zu passieren sind und eine exakt vorbestimmte Position zu erreichen ist.

Da bereits das Einführen des Führungsdrahtes in das zu seiner Aufnahme vorgesehene Lumen im entsprechenden Katheter bzw. der Elektrodenleitung eine diffizile Angelegenheit ist, wurden spezielle Einführhilfen entwickelt, die es dem Operateur erleichtern, das proximale Ende des Katheters bzw. der Elektrodenleitung mit dem Führungsdraht zu treffen. Derartige Einführhilfen sind in ihrer Gestalt auf die spezielle Steckerform der Elektrodenleitung abgestimmt und passen daher jeweils nur zu einer bestimmten Elektrodenleitung. Darüber hinaus ist aus der US 6,652,492 B1 eine durchgehend geschlitzte Einführhilfe bekannt geworden. Diese Einführhilfe ist in nachteiliger Weise während der Handhabung durch den Operateur - die oftmals ein mehrfaches Einführen des Führungsdrahtes in die Elektrodenleitung erfordert - verlierbar und muss dann durch eine neue sterile Einführhilfe ersetzt werden. Das bringt Unruhe in den Operationsablauf und erfordert natürlich auch zusätzlichen logistischen Aufwand.

Die Dokumente US 2008/0082051 A1, US 2006/0116691 A1, US 2004/0073193 A1, US 6,110,146 offenbaren ebenfalls nur durchgehende Schlitze in den Führungsdraht-Einführhilfen.

Der Erfindung liegt daher die Aufgabe zugrunde, eine verbesserte Einführhilfe der oben genannten Art bereitzustellen, die sich insbesondere durch leichte und sichere Handhabbarkeit, insbesondere der Unterbindung der Verlierbarkeit, und Anwendbarkeit für verschiedene Typen von Kathetern und Elektrodenleitungen auszeichnet.

Diese Aufgabe wird durch eine Einführhilfe mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, die Einführhilfe als sich auf verschiedene proximale Enden von Kathetern oder Elektrodenleitungen in gewissem Rahmen selbsttätig anpassendes Bauteil auszuführen. Sie schließt weiter den Gedanken ein, den Fixierungsabschnitt der Einführhilfe zu diesem Zweck radial elastisch aufweitbar zu gestalten. Schließlich gehört zur Erfindung der Gedanke, im Fixierungsabschnitt mindestens über den größeren Teil von dessen Längserstreckung einen Schlitz vorzusehen, der die vorgesehene Aufweitbarkeit, verbunden mit hinreichender Haltekraft auf dem Katheter- bzw- Leitungsende, teilweise erbringt. Weiterhin gehört zur Erfindung der Gedanke, die Innenwandung des Fixierungsabschnittes frei von (Ring-)Kanten, Absätzen etc. zu halten, was wiederum durch die elastische Aufweitbarkeit und das Vorsehen des Schlitzes erst ermöglicht oder zumindest begünstigt wird. Schließlich gehört zur Erfindung der Gedanke, dass der vorgesehene Schlitz nur im Fixierungsabschnitt verläuft, und nicht über diesen hinaus geht.

Die vorgeschlagene Lösung ist insbesondere im Hinblick auf folgende Anwendungsaspekte vorteilhaft:
- Während der Implantation einer Elektrode wird die Einführhilfe mehrmals auf den Steckerpin gesteckt, um etwa ein Führungsdraht- bzw. Mandrinwechsel (härtere Mandrins, vorgebogene Mandrins) vorzunehmen.
- Ebenfalls erforderlich ist ein häufiges Abziehen der Einführhilfe vom Steckerpin um etwa eine Elektrode im Patienten einmessen zu können. Dabei ist es von Vorteil, dass die Einführhilfe wegen des nicht durchgängigen Schlitzes nicht aus dem Implantationssystem entfernt werden, d.h. nicht vom Mandrin abgenommen werden oder in die Operationswunde fallen kann. Sie bleibt dort, wo man sie braucht.
- Beim Fixieren von "Schraubenelektroden" wird die Einführhilfe einfach vom Steckerpin abgezogen und bleibt auf dem überstehenden Mandrin hängen. Somit kann auf den Steckerpin ein Schraubwerkzeug gesetzt werden und es ist gewährleistet, dass der Mandrin die Elektrode während der Fixierung auch gegen die Herzwand anstemmen kann.
- Dank der elastischen Aufweitbarkeit kann die Einführhilfe trotz der maßlichen Unterschiede verschiedener (Stecker-)Anschlüsse, zu denen sie passen soll, auf diesen hinreichend sicher halten.
- Die im Wesentlichen "glatte" Ausführung der Innenwandung des Abschnitts, ohne innere Kanten oder Absätze, gewährleistet, dass auf den Steckeranschlüssen angebrachte Farbmarkierungen während der für die Implantation erforderlichen Handhabungen unbeschädigt bleiben.

Aus den vorstehend genannten technischen Vorteilen ergeben sich unter anderem folgende wirtschaftliche Vorteile:
- Es kann für Elektrodenleitungen bzw. Katheter mit unterschiedlichen Anschlüssen eine Einführhilfe mit einer einzigen Ausführung entwickelt, produziert und geliefert werden, was die Herstellungs-, Vertriebs- und Logistikkosten auf Herstellerseite senkt.
- Auf Anwenderseite ergibt sich eine entsprechende Vereinfachung bei der Lagerhaltung und Operationsvorbereitung, und auch der Anwender profitiert von den geringeren Entwicklungs- und Herstellungskosten.

In einer Ausführung der Erfindung besteht mindestens der geschlitzte Teil des Fixierungsabschnitts aus einem formelastischen Kunststoff. In einer weiteren Ausgestaltung ist die Einführhilfe insgesamt einstückig aus einem in gewissem Grade formelastischen Kunststoff ausgeführt.

Alternativ hierzu ist eine Ausführung möglich, bei der im geschlitzten Teil des Fixierungsabschnitts ein radial wirkendes Federelement vorgesehen ist. Eine solche Ausführung ist zwar etwas aufwändiger herzustellen, ist in ihren mechanischen Eigenschaften aber weitgehend unabhängig vom Einsatz bestimmter Kunststoffmaterialien zu entwerfen.

In einer weiteren Ausführung der vorgeschlagenen Führungsdraht-Einführhilfe weist der Fixierungsabschnitt einen längs verlaufenden Schlitz auf. Andererseits kann vorgesehen sein, dass der Fixierungsabschnitt einen spiralig verlaufenden Schlitz aufweist. Schließlich kann in weiteren Ausführungen der Fixierungsabschnitt auch mehrere Schlitze aufweisen, die gegebenenfalls unterschiedliche Dimensionen und/oder verschiedene Gestalt haben.

In einer weiteren Ausführung der Erfindung ist die Innenwandung des Fixierungsabschnitts proximal durch einen Anschlag für das eingeschobene Ende des Katheters oder der Elektrodenleitung begrenzt.

Bevorzugt ist der Innendurchmesser des Aufweitungsabschnitts an seinem dem Fixierungsabschnitt benachbarten Ende kleiner als der Innendurchmesser des Fixierungsabschnitts, und zwar vorteilhafterweise abgestimmt auch den Durchmesser eines im Katheter bzw. in der Elektrodenleitung vorgesehenen Lumens zur Aufnahme des Führungsdrahtes. Der Anschlag ist somit durch das Ende des Aufweitungsabschnitts gebildet.

Eine weitere Ausführung der Erfindung sieht vor, dass der Innendurchmesser des Fixierungsabschnitts (mit geringfügigem Untermaß) auf den Stecker einer Norm-Elektrodenleitung, insbesondere einen DF-4- und/oder IS-1- und/oder IS-4- und/oder DF-1-Anschluss, abgestimmt ist. Neben den genannten Normanschlüssen kann die Einführhilfe bedarfsweise auch auf weitere Anschlüsse passfähig gestaltet sein.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine perspektivische Ansicht einer erfindungsgemäßen Führungsdraht-Einführhilfe,
- Fig. 2A und 2B: eine Draufsicht (vom freien Ende des Fixierungsabschnitts) und eine Längsschnittdarstellung der Führungsdraht-Einführhilfe nach Fig. 1 und
- Fig. 3: eine Darstellung zur Verdeutlichung des Einsatzes der Führungsdraht-Einführhilfe nach Fig. 1 und 2A/2B.

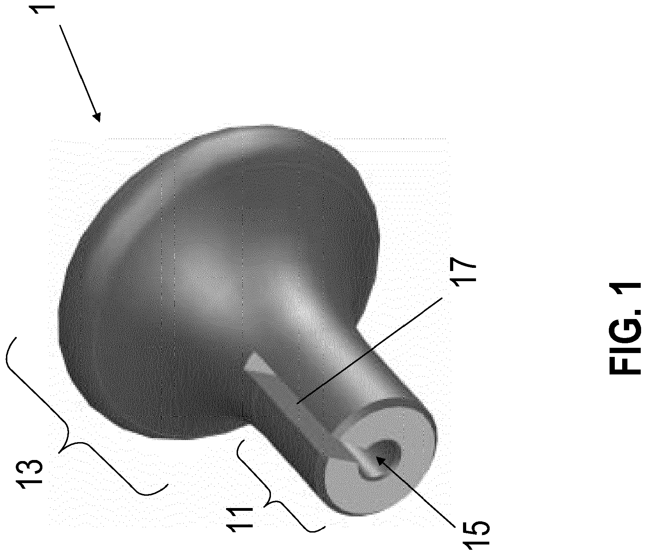

Fig. 1 zeigt in perspektivischer Darstellung eine aus einem formelastischen Kunststoff einstückig hergestellte Führungsdraht-Einführhilfe 1, deren Grundform diejenige eines Horns ist und die einen Fixierungsabschnitt 11 und einen sich an diesen anschließenden Aufweitungsabschnitt 13 sowie eine zentral längs durchgehende Öffnung 15 aufweist. Zu erkennen ist außerdem ein sich über fast die gesamte Länge des Fixierungsabschnittes 11 erstreckender, längs verlaufender Schlitz 17. Weiterhin ist zu erkennen, dass der Aufweitungsabschnitt keinen Schlitz aufweist.

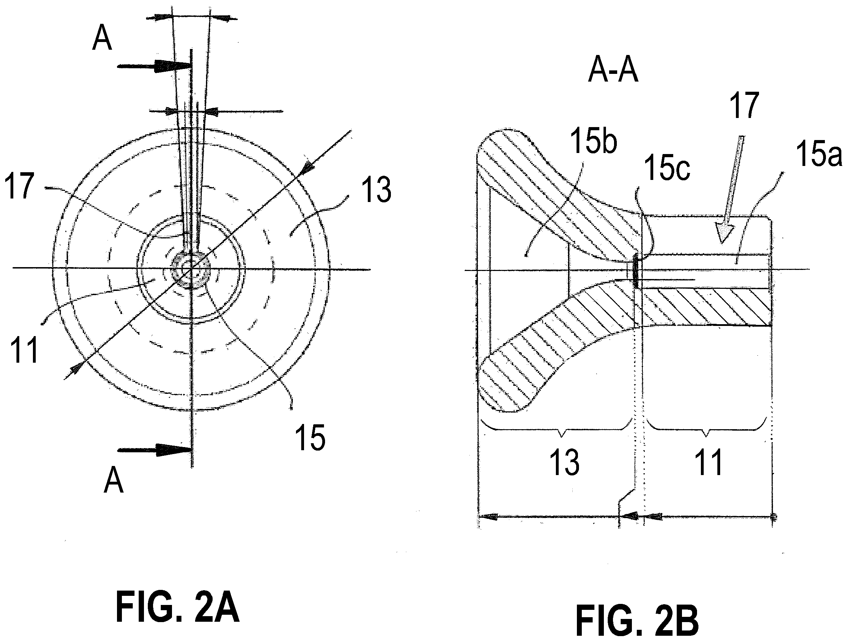

Die Figuren 2A und 2B zeigen diesen Aufbau in einer Draufsicht bzw. Längsschnittdarstellung etwas detaillierter. Hier ist insbesondere auch zu erkennen, dass die durchgehende zentrische Öffnung 15 im Fixierungsabschnitt 11 einen ersten, zylindrischen Teilabschnitt 15a mit konturloser Wandung und im Aufweitungsabschnitt 13 einen zweiten, sich hornförmig aufweitenden Abschnitt 15b umfasst. An der Grenze zum benachbarten Ende des Fixierungsabschnitts 15a ist dessen Durchmesser geringer als derjenige des ersten Abschnitts 15a, so dass hier ein ringförmiger Anschlag 15c gebildet ist. Diese Ausgestaltung der durchgehenden zentralen Öffnung 15 dient einerseits zur Begrenzung der Einschubtiefe eines in den Fixierungsabschnitt einzuschiebenden Elektrodenleitungssteckers oder Katheterendes und andererseits dazu, einen aus dem weiten Ende des Aufweitungsabschnitts 13 eingeführten Führungsdraht sicher in ein für ihn vorgesehenes Lumen in der Elektrodenleitung bzw. dem Katheter zu lenken.

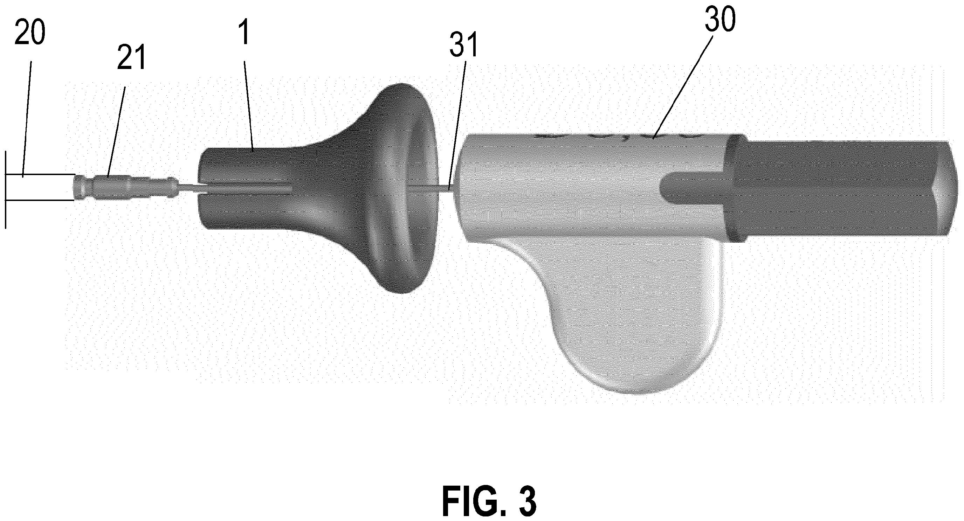

Fig. 3 zeigt die Führungsdraht-Einführhilfe 1 im Einsatz an einem Steckeranschluss 21 einer Elektrodenleitung 20 mit eingeführtem Führungsdraht 31 einer Führungsdrahtanordnung 30.

Die Ausführung ist nicht auf dieses Beispiel und die oben hervorgehobenen Aspekte beschränkt, sondern ebenso in einer Vielzahl von Abwandlungen möglich, die im Rahmen fachgemäßen Handelns liegen. So sind insbesondere andere Formgestaltungen des Aufweitungsabschnitts der Einführhilfe (darunter insbesondere eine Trichterform mit kegelstumpfförmiger Wandung), mehrteilige Ausführungen und vielfältige Variationen hinsichtlich der longitudinalen und lateralen Abmessungen, Wandungsstärke etc. als im Bereich der Erfindung liegend anzusehen.

## Patentansprüche

1. Führungsdraht-Einführhilfe (1) zum Aufsetzen auf ein proximales Ende eines Katheters oder einer implantierbaren Elektrodenleitung und zur Erleichterung der Einführung eines Führungsdrahtes,
ausgebildet als horn- oder trichterförmiger Körper mit einem rohrförmigen Fixierungsabschnitt (11) und einem von diesem ausgehenden Aufweitungsabschnitt (13), wobei der Fixierungsabschnitt (11) eine konturlose Innenwandung aufweist und mindestens über den größeren Teil seiner Längserstreckung geschlitzt und elastisch aufweitbar ist, **dadurch gekennzeichnet, dass** der Schlitz (17) nur im Fixierungsabschnitt (11) der Einführhilfe (1) vorgesehen ist.

2. Führungsdraht-Einführhilfe (1) nach Anspruch 1, wobei mindestens der geschlitzte Teil des Fixierungsabschnitts (11) aus einem formelastischen Kunststoff besteht.

3. Führungsdraht-Einführhilfe (1) nach Anspruch 2, einstückig ausgeführt aus einem formelastischen Kunststoff.

4. Führungsdraht-Einführhilfe (1) nach einem der vorangehenden Ansprüche, wobei im geschlitzten Teil des Fixierungsabschnitts (11) ein radial wirkendes Federelement vorgesehen ist.

5. Führungsdraht-Einführhilfe (1) nach einem der vorangehenden Ansprüche, wobei der Fixierungsabschnitt (11) einen längs verlaufenden Schlitz (17) aufweist.

6. Führungsdraht-Einführhilfe (1) nach einem der vorangehenden Ansprüche, wobei der Fixierungsabschnitt (11) einen spiralig verlaufenden Schlitz aufweist.

7. Führungsdraht-Einführhilfe (1) nach einem der vorangehenden Ansprüche, wobei die Innenwandung des Fixierungsabschnitts proximal durch einen Anschlag (15c) für das eingeschobene Ende des Katheters oder der Elektrodenleitung begrenzt ist.

8. Führungsdraht-Einführhilfe (1) nach Anspruch 7, wobei der Innendurchmesser des Aufweitungsabschnitts (13) an seinem dem Fixierungsabschnitt (11) benachbarten Ende kleiner als der Innendurchmesser des Fixierungsabschnitts (11) und der Anschlag (15c) somit durch das Ende des Aufweitungsabschnitts (13) gebildet ist.

9. Führungsdraht-Einführhilfe (1) nach einem der vorangehenden Ansprüche, wobei der Innendurchmesser des Fixierungsabschnitts (11) untermaßig auf den Stecker (21) einer Norm-Elektrodenleitung (20), insbesondere einen DF-4- und/oder IS-1- und/oder IS-4- und/oder DF-1-Stecker, abgestimmt ist.

## Claims

1. A guide wire insertion aid (1) for attaching onto a proximal end of a catheter or an implantable electrode lead and for facilitating the insertion of a guide wire,
designed as a horn- or funnel-shaped body comprising a tubular fixing section (11) and an expanding section (13) extending therefrom, the fixing section (11) having an inner wall without contour and being slotted and elastically expandable at least across the majority of the longitudinal extension thereof, **characterized in that** the slot (17) is only provided in the fixing section (11) of the insertion aid (1).

2. The guide wire insertion aid (1) according to claim 1, wherein at least the slotted portion of the fixing section (11) is made of a shape-elastic plastic material.

3. The guide wire insertion aid (1) according to claim 2, designed in one piece from a shape-elastic plastic material.

4. The guide wire insertion aid (1) according to any one of the preceding claims, wherein a radially acting spring element is provided in the slotted portion of the fixing section (11).

5. The guide wire insertion aid (1) according to any one of the preceding claims, wherein the fixing section (11) includes a longitudinally extending slot (17).

6. The guide wire insertion aid (1) according to any one of the preceding claims, wherein the fixing section (11) includes a spirally extending slot.

7. The guide wire insertion aid (1) according to any one of the preceding claims, wherein the inner wall of the fixing section is proximally delimited by a stop (15c) for the inserted end of the catheter or the electrode lead.

8. The guide wire insertion aid (1) according to claim 7, wherein the inner diameter of the expanding section (13), at the end thereof adjacent to the fixing section (11), is smaller than the inner diameter of the fixing section (11), and the stop (15c) is thus formed by the end of the expanding section (13).

9. The guide wire insertion aid (1) according to any one of the preceding claims, wherein the inner diameter of the fixing section (11) is matched in an undersized dimension to the connector (21) of a standard electrode lead (20), in particular a DF-4 and/or IS-1 and/or IS-4 and/or DF-1 connector.

## Revendications

1. Aide à l'insertion d'un fil de guidage (1) à mettre en place sur une extrémité proximale d'un cathéter ou d'une ligne d'électrode implantable et permettant de faciliter l'insertion d'un fil de guidage,
conçue sous forme d'un corps en forme de corne ou d'entonnoir avec un segment de fixation (11) de forme tubulaire et un segment d'élargissement (13) partant de celui-ci,
où le segment de fixation (11) présente une paroi intérieure sans contour et est au moins fendu sur la plus grande partie de son extension longitudinale et peut être élargi de manière élastique, **caractérisée en ce que** la fente (17) est prévue uniquement dans le segment de fixation (11) de l'aide à l'insertion (1).

2. Aide à l'insertion d'un fil de guidage (1) selon la revendication 1, dans laquelle au moins la partie fendue du segment de fixation (11) est constituée d'une matière synthétique élastique.

3. Aide à l'insertion d'un fil de guidage (1) selon la revendication 2, conçue dans une matière synthétique élastique.

4. Aide à l'insertion d'un fil de guidage (1) selon l'une des revendications précédentes, dans laquelle un élément élastique agissant radialement est prévu dans la partie fendue du segment de fixation (11).

5. Aide à l'insertion d'un fil de guidage (1) selon l'une des revendications précédentes, dans laquelle le segment de fixation (11) présente une fente (17) s'étendant longitudinalement.

6. Aide à l'insertion d'un fil de guidage (1) selon l'une des revendications précédentes, dans laquelle le segment de fixation (11) présente une fente s'étendant sous forme d'une spirale.

7. Aide à l'insertion d'un fil de guidage (1) selon l'une des revendications précédentes, dans laquelle la paroi intérieure du segment de fixation est limitée de manière proximale par une butée (15c) pour l'extrémité insérée du cathéter ou de la ligne d'électrode.

8. Aide à l'insertion d'un fil de guidage (1) selon la revendication 7, dans laquelle le diamètre intérieur du segment d'élargissement (13) est conçu plus petit au niveau de son extrémité à proximité du segment de fixation (11) que le diamètre intérieur du segment de fixation (11), et la butée (15c) est ainsi formée par l'extrémité du segment d'élargissement (13).

9. Aide à l'insertion d'un fil de guidage (1) selon l'une des revendications précédentes, dans laquelle le diamètre intérieur du segment de fixation (11) est adapté trop petit au connecteur (21) d'une ligne d'électrode (20) normalisée, notamment un connecteur DF-4, et/ou un connecteur IS-1, et/ou un connecteur IS-4, et/ou un connecteur DF-1.
